# EUROPEAN PATENT APPLICATION

(11) **EP 2 716 689 A1**
(43) Date of publication of application: **09.04.2014**
(21) Application number: 12187515.7
(22) Date of filing: 05.10.2012
(51) Int. Cl.: C08G 83/00, C08J 3/075, C08J 3/24, C08F 16/24, C08F 16/26, C08F 16/28, C08F 16/30, A61K 47/48, C08F 220/38

(54) **Polymer comprising a plurality of branches having at least one disulfide group and/or at least one vinyl group**

(71) Applicant: National University of Ireland, Galway, Galway (IE)
(72) Inventor: Wang, Wenxin, Galway (IE); Aied, Ahmed, Galway (IE); Pandit, Abhay, Galway (IE)
(74) Representative: Gates, Marie Christina Esther

(57) **Abstract**

A polymer for transfecting a nucleic acid into a cell, the polymer comprising a plurality of polymeric branches, wherein at least one branch comprises at least one disulfide group and at least one vinyl group, which may an unsubstituted vinyl or a functionalized vinyl group.

## Description

### Field of the Invention:

The invention relates to the provision of hyperbranched polymers which may be used as mucleic acid transfection reagents. In particular, biodegradable hyperbranched polymers are provided and can be used in gene delivery.

### Background to the Invention:

Gene therapy promises to be one of the most important frontiers in medicine. Although there have been some major setbacks in the treatment of disease, many new systems show great potential for effective and safe treatment of genetic related diseases. The demand for reagent-based genetic transfection systems has expanded rapidly in the last decade, currently standing at $250M per year and rising.

Evidence of growth in this technology can be seen from the rise in the number of viral based gene therapy companies (from 44 in 1995 to more than 190 at present), major financing agreements since 2004 with well over $100 M in funding, and significant progress in non-viral based gene therapy systems. There are now a range of research trials beginning and ongoing in both viral and non-viral based therapies, with viral trials ranging from *"Determining a viral load threshold for treating Cytomelovirus (CMV)"to "Viral respiratory and gastrointestinal infections in children under 6 years of* age". The variety of viral research alone covers numerous diseases including HIV, hepatitis, herpes virus, influenza and heptocellular carcinoma. Use of viral vector have raised concerns in terms of immunogenicity, pathogenicity and oncogenicity. Viruses are limited in the size of DNA that can be transfected.

Non-viral transfection agents overcome many concerns associated with viral vector use, although there are typically problems associated with lower transfection success. In the non-viral based therapies, there is a wider range of genetic diseases being studied. Cystic fibrosis, hepatitis, chronic heart disease, diabetes mellitus, chronic pulmonary disease as well as influenza virus, HIV and nasopharyngeal carcinoma are the current and ongoing trials with a variety of drug interventions being used.

There are a range of in vitro and in vivo transfection reagents currently on the market to suit the needs of many areas of study. Altogen Biosystems carry a range of in vivo reagents that are siRNA and have a plasmid DNA delivery reagent, which are lipid based, polymer based, nano-particle based and PEGliposme based: all of which have been functionally tested on mice, have efficient delivery to pancreas, liver, kidney, spleen and certain tumor types with minimal toxicity. Thermo Scientific carry two *in vivo* transfection reagents: ExGen 500 and TurboFect which provide gene delivery via multiple routes of administration and reproducible results. Thermo Scientific also provide in vitro transfection reagents with minimal cytotoxicity, ready-to-use reagents with high transfection efficiency on a wide variety of cell types. Polyplus carry JetPEI DNA which is compatible with serum and antibiotics, has a broad cell-line spectrum and a straightforward protocol. InvivoGen claims to have the first lyophilized-based cationic lipid-based transfection reagent, LyoVec. It is rapid and easy to use with an optimized procedure and a long shelf-life. BioMol has ExGen 500 which is capable of transfecting a wide variety of cells. Pierce Protein provides optimum delivery in 3 to 4 hours after incubation. SignaGen Laboratories have PepJet DNA and GenJet DNA which claim to have a high in vivo transfection efficiency and give good renilla luciferase expression in lung, liver, spleen and kidney. T-Pro Biotechnology provide the T-Pro G-Fect Transfection Reagent which is suitable for DNA administration via various routes but the DNA quality is critical for successful transfection. Reagents such as Lipofectamine^{™}2000, Polyethyleneimine,

SuperFect and FuGENE® are now hugely important tools for R&D scientists. Some other companies and their products include INVITOGEN: Lipofectin, Liptofectamine, Lipofectamine Plus and Lipofectamine 2000; CLONTECH: Clonefectin; STRATAGENE: GeneJammer, LipoTAXI; GENE THERAPY SYSTEMS: GenePORTER. Indeed, Lipofectamine® reagents, owned by Life Technologies, have become the most referenced transfection reagents with over 42,000 citations to date. Despite market saturation, limitations in terms of transfection efficiency, cellular toxicity and clinical application mean the improvement drive is ongoing.

The advantages of non-viral polymer/liposome transfection reagents is that they consist of simple components, many are commercially available and they exhibit fewer biosafety problems.

Even though significant improvements have occurred over the past decade in the field of non-viral gene therapy, the efficiency is still lacking with many new technologies unable to move beyond the stage of clinical trials and FDA approval.

Furthermore, the translation of 'Gene Therapy' developments to clinical application has to date been severely restricted by mutagenesis concerns with virus-based delivery systems and efficiency issues with currently available chemical-based reagents.

It is an object of the invention to provide effective DNA transfection non-viral vectors which will facilitate new treatments of genetic diseases and will improve the production of therapeutic proteins.

### Statements of the Invention

Accordingly to the present invention as set out in the appended claims, there is provided a polymer for transfecting a nucleic acid into a cell, the polymer comprising a plurality of branches, wherein at least one of the branches comprises: at least one disulfide group, and/or at least one vinyl group, which may an unsubstituted vinyl or a functionalized vinyl group, and/or combinations thereof.

Preferably, at least one of the plurality of polymeric branches comprises at least one ionisable amine, diamine, tri-amine or amino alcohol functionality, or combinations thereof.

Suitably, the vinyl group or vinyl functionality of the polymer may be a methacrylate group which can be unsubstituted or substituted with a linear or branched C₁₋₆ alkyl. Methyl and ethyl substituents are preferred.

Preferably, the polymer branches comprise more than one, and preferably a plurality of disulfide and/or vinyl groups or vinyl functionalities in each branch.

Suitable vinyl functionalisations include primary, secondary or tertiary amine, such as diamine or amino alcohol.

Suitably, in the polymer of the invention, each of the plurality of polymeric branches comprises an alkylene based chain from which further alkylene based chains split off to form further branches.

Desirably, at least one of the plurality of polymeric branches comprises at least one hydroxyl functionality.

Suitably, at least one of the plurality of polymeric branches comprises at least one of an ester, ether, thioate or amide functionality.

In the polymer of the invention, the at least one of the each of the plurality of polymeric branches comprises at least one halide selected from the group consisting of: F, Cl, Br and I.

It is preferred that the branches terminate with a halide. Terminal or terminating halides on the branches are preferred. Branches terminating in Br are particularly preferred.

In a preferred embodiment, the polymer is hyperbranched.

In one arrangement, the polymer of the invention can adopt a knotted polymer structure, which can be single cyclized or multi-cyclized.

Desirably, the polymer of the invention has low polydispersity index meaning that there is little or no difference between the number average and weight average molecualer weight of the polymer as determined by Gel Premeation Chromatography.

In a preferred embodiment, the alkyl based chain of the polymer may be an ethylene chain which may be unsubstituted or substituted with a linear or branched C₁₋₆ alkyl. Unsubstituted ethylene chains are particularly preferred.

In a particularly preferred embodiment, the polymer of the invention is hyperbranched and comprises a plurality of branched alkylene chains, wherein at least one of the alkylene branches comprises at least one disulfide group in the alkylene chain, and the alkylene sidechain is further substituted with at least one vinyl group or vinyl functionality; and wherein at least one of the alkylene branches terminates with at least one of a carbocation, a halide, an ionisable amine, diamine and a hydroxyl group.

In a preferred embodiment there is provided a polymer for transfecting nucleic acid into a cell comprising at least one polymeric ethylene backbone having:
at least one first sidechain repeating unit,
   wherein one end of each sidechain is covalently bonded to the ethylene backbone by an ester, amide or thioate linkage, and wherein the other end of each first sidechain comprises at least one of a vinyl group, a hydroxide, a halide, an amine group or a diamine group to form a polymer or comprises at least one an ester, amide or thioate linkage to a polymeric ethylene backbone (which can be same or different) to form a branched polymer having at least two polymeric ethylene backbones, and wherein each of the first sidechains further comprises at least one disulfide group located between either ends of the first sidechain repeating unit; and
at least one second sidechain repeating unit,
wherein one end of each sidechain is covalently bonded to the at least one polymer ethylene backbone by an ester, amide or thioate linkage, and wherein the other end of each second sidechain comprises with an ionisable amine or a diamine group to form an polyelectrolyte for complexing nucleic acid.

In a preferred embodiment, the polymer further comprises a plurality of additional polymer backbone chains having the at least one first sidechain repeating unit and the at least one second sidechain repeating unit as defined above, the plurality of additional polymer backbone chains being arranged in a branched polymeric repeating pattern to form a hyperbranched polymer.

Thus the invention provides a linear, branched or hyperbranched polymer having controlled molecular weights and narrow molecular weight distributions. Suitably, the molecular weight, and the composition (i.e. the number and ratio) of first and second sidearm chains can be controlled or adjusted by appropriate choice of the co-monomer composition used to provide the polymer (for example, by a RAFT, ATRP or DE-ATRP or other controlled living polymerization process. Desirably, quaternisation reaction of the polymer with reagents such as methyl iodide can leads to watersoluble cationic polyelectrolytes with linear, branched or hyperbranched structures. The polymers of the invention are a significant achievement as it is known in the art that synthesis of well-defined branched polymers with ionic or ionisable groups is difficult. The polymer of the invention is important as it can be used as a non-viral gene transfection agent. The polymer of the invention has been shown to have excellent transfection efficiency, low cytotoxicity and is stable in nature. The polymer (plus nucleic acid-polyplex) displays fast intracellular degradation, and can be used in transfection of a broad range of cell types.

In a preferred embodiment, ester linkages are preferred. Suitably, the polymer ethylene backbone comprises at least one halide and optionally at least -OH. Suitably, halide selected from F, Cl, Br or I. The ethylene backbone chain of the polymer may be substituted with other alkyl C₁ to C₆ alkyl groups (preferably methyl, ethyl or propyl) or may be unsubstituted (apart from the sidearm chains mentioned above). Desirably, the polymer may be linear, linearly branched, branched or hyperbranched. Preferably, the polymer comprises more than one backbone chain in a hyperbranched type structure. Desirably, the polymer comprises a plurality of backbone chains, one or more of which are covalently linked together by each end of at least one of the first sidearm chains. This arrangement gives rise to the hyperbranched structure. The degree of crosslinking provides for formation of a branched or hyperbranched structure.

Desirably, the at least one linear polymeric ethylene backbone and/or the additional polymer backbone chain(s) further comprises at least one initiator sidechain, which arises from the polymerisation initator used to prepare the polymer of the invention. One end of the initiator sidechain may be covalently bonded to the ethylene backbone by an ester, amide or thioate linkage and wherein the other end of the initiator sidechain terminates with C₁ to C₆ alkyl group.

Suitably, the amine group may be a primary, a secondary or a tertiary amine group or a diamine group of general formula -NH-alkyl-NH₂, wherein the alkyl group is a linear or branched alkyl group having from 1 to 6 carbon atoms, and which can be substituted or unsubstituted with C₁₋₆ alkyl.

Desirably, the amine group or diamine groups comprise cationic amine groups or amine groups that become cationic under certain conditions, i.e., ionisable. Suitably such conditions include physiological conditions. Particularly preferred are tertiary amines, as they are easily protonated at acidic endosomal pH, and therefore assist more in disruption of the endosome to allow movement of the polymer into the cell.

In a particularly preferred embodiment, in the polymer of the invention the terminating vinyl group may be provided as a methacrylate group which can be unsubstituted or substituted with C₁₋₆ alkyl. The vinyl group may also be provided as C₁ - C₆ alkylmethacrylate, for example, methyl, ethyl propyl, butyl, pentyl or hexyl methacrylate group. The alkyl groups in these instances may be substituted or unsubstituted with at least one methyl or ethyl group.

In a preferred embodiment, the polymer comprises at least one repeating unit selected from:

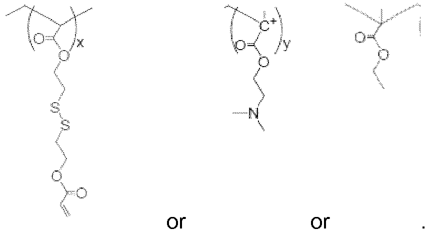

In a preferred embodiment, the polymer comprises at least one repeating unit selected from the group consisting of 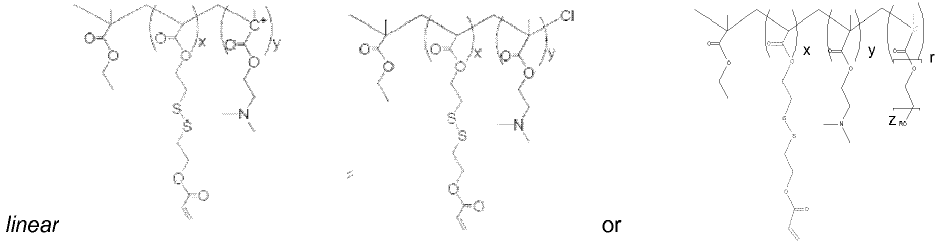 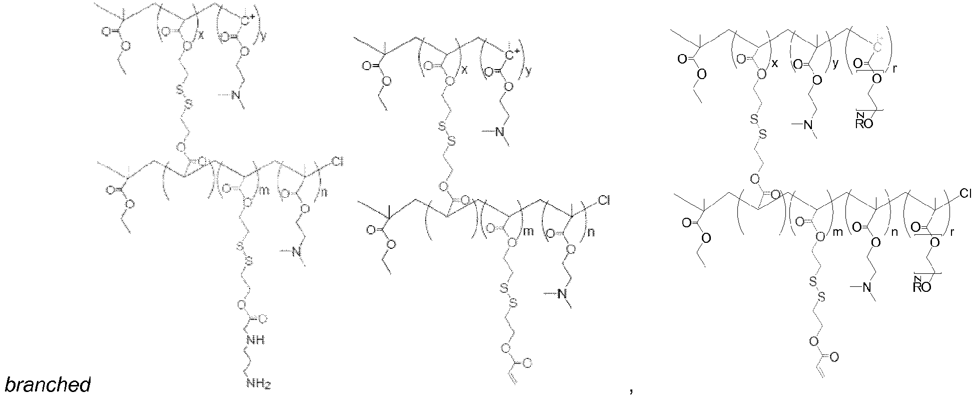 wherein m is X and n is greater than 1, wherein the structure above forms either one polymer unit or form the basis for the repeating unit of a hyperbranched polymer. The skilled person will appreciate, that the terminating vinyl group of second sidearm chain in the polymer can form a covalent bond with a further still back bond chain to further grow the polymer.

In one aspect, the polymer of the invention may be used as a nucleic acid or a drug delivery agent or as a transfecting agent in non-viral gene delivery or as a polyelectrolyte for complexing a nucleic acid or in an analytical application in bio-imaging techniques for tagging proteins/nucleic acids inside the cell.

In nucleic acid or drug delivery embodiments, the polymer can be used in the treatment of a disease that can be treated by genetic/nucleic acid manipulation. By genetic/nucleic acid manipulation, it is such where transient expression of genetic entities such as DNA, RNA, aptamers, etc is facilitated, that is, where the delivered agent enters the cell and functions without being integrating into the host's genome.

Examples of such diseases include nucleus pulpous, Parkinson's disease, cardiovascular disease and recessive dystrophic epidermolysis bullosa.

It will be understood that drug as used herein can mean a chemical entities such as a pharmaceuticals; proteins including peptides, polypeptides and/or glycopeptides; DNA and/or RNA (nucleic acids); aptamers (nucleic acids); and or nucleic acid products such as antibodies and/or antibody fragments.

Thus, in a related aspect of the invention, the polymer may be used as a medicament delivery agent. The polymer of the invention is capable of self-assembly with nucleic acid, for example DNA, to form a polyplex. Polymer embodiments having free cationic amine groups are particularly desirable since they readily form polyplexes with nucleic acids.

When the polyplex of the invention is applied to cultured cells, the polyplex are suitable for entry into the cell with the nucleic acid is released on degradation of the polymer, and thereby gets integrated into the host cell genome. A desired protein expression is thereby achieved. Thus the polymers of the invention are suitable gene or gene product a drug delivery agent or medicament delivery agents or as a transfecting agent in non-viral gene delivery.

In a related aspect, the polymer of the invention may be used as a transfecting agent in a method of non-viral gene delivery. Accordingly, there is provided a method of transfecting an cell, comprising contacting the cell with a transfection agent comprising a hyperbranched polymer comprising a plurality of disulfide groups and a plurality of vinyl groups or vinyl functionalities, wherein the step of contacting the least one comprising nucleic acid and a polycationic compound transfects the cell with the nucleic acid.

Suitable vinyl functionalisations include primary, secondary or tertiary amine, such as diamine or amino alcohols.

Suitably, the cell is an animal cell. Desirably animal cell is mammalian cells. Preferably, the cell is a human cell.

The transfection may optionally be carried out in the presence of serum. Suitably, the serum may be fetal bovine serum or human serum. The transfection may be carried out with a lipid aggregate in the presence of the polymer transfection agent of the invention. Typically the nucleic acid and/or the polymer transfection agent may be provided in the lipid aggregate. When a cell is contact with such an aggregate the cell is transfected with the nucleic acid in question. For example, the hyperbranched polymer may be provided as part of the lipid aggregate.

Desirably, the nucleic acid is DNA or RNA or derivatives thereof, or combinations thereof.

In a related aspect, there is provided a process for synthesising a hyperbranched polymer comprising polymerising a divinyl disulfide monomer with other optional polymerisable monomers and/or initiators under suitable polymerisation conditions to effect polymerisation of the monomer by a living polymerisation process.

Suitably, the polymerisaton conditions are provided by use of an RAFT, ATRP or a DE-ATRP or other controlled living polymerisation process.

In a further related aspect still, there is provided a method of manufacturing a polymer for transfecting agent for non-viral gene delivery comprising radial polymerization of at least one multi-vinyl monomer or co-monomer, or combinations thereof, using a controlled living polymerization process in which
polymerization is initiated by a radical initiator in the presence of a catalyst;
subsequent polymerization of the multi vinyl monomers to form at least one first linear polymer backbone chain terminating in a halide or other leaving group;
optional further polymerization of the multi vinyl monomers with a second linear polymer backbone chain terminating in a halide or other leaving group to form a branched or hyperbranched polymer;
optional vinyl group termination by conjugate addition of a terminating group.

Terminating group is preferably a diamine.

In a preferred embodiment, the process of the invention uses a divinyl disulfide monomer is selected from the group consisting of: 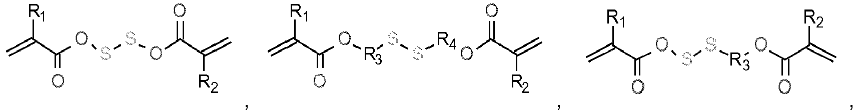 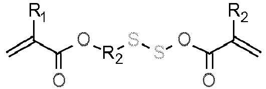 in which, independently, R₁, R₂, R₃ and R₄ can be C₀ - C₆ alkyl.

Particularly preferred alkyl groups are methyl, ethyl, propyl or butyl groups, which can be linear or branched. A preferred alkyl group is ethyl.

n a preferred embodiment, R₁ and R₂ are both unsubstituted (i.e. C₀, wherein a pair of -H are found on the terminal end of the alkene bond. Examples of this embodiment include:

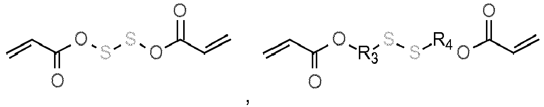

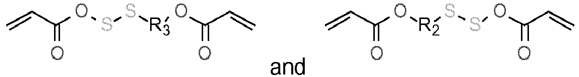

In this embodiment, independently R₃ and R₄ can be C₀ - C₆ alkyl. Particularly preferred alkyl groups are methyl, ethyl, propyl or butyl groups, which can be linear or branched. A preferred alkyl group is ethyl.

In a further preferred embodiment, either one of R₃ or R₄ may be ethyl. Examples include:

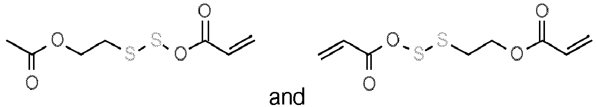

In a further preferred embodiment, either one of R₃ or R₄ may be ethyl, propyl, butyl or pentyl. In this embodiment, the disulfide group can be centrally positioned or preferentially on toward one or the other ends of the monomer chain. In another embodiment, more than one disulfide group can be provided in the monomer chain. Examples include:

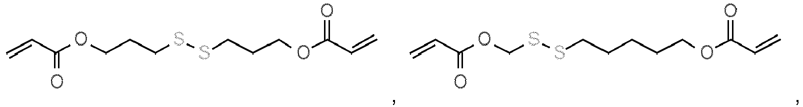

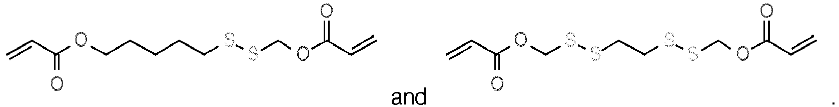

In a particularly preferred embodiment, there is provided a multifunctional vinyl monomer having structure:

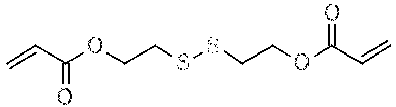

The IUPAC name for this compound is 2-(2-prop-2-enoyloxyethyldisulfanyl)ethyl prop-2-enoate.

Desirably, the divinyl disulfide monomer used in the polymerisation process of the invention is

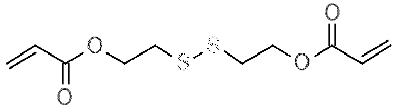

In a related aspect, there is provided a monomer compound having general formula:

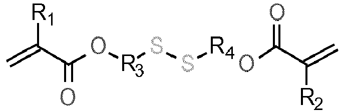

in which, independently, R₁, R₂, R₃ and R₄ can be C₀ - C₆ alkyl. Particularly preferred alkyl groups are methyl, ethyl, propyl or butyl groups, which can be linear or branched. A preferred alkyl group is ethyl.

Suitably, the monomer has the structure:

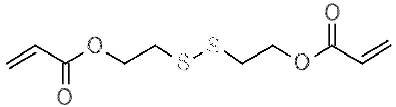

Desirably, this compound may be used in a living polymerisation process.

Suitably, the living polymerisation process is a RAFT, an ATRP or a DE-ARTP process.

Suitably, at least one co-vinyl monomers may be used in the process of the invention may have the general structure:

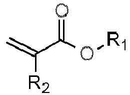

wherein R₁ is C₁- C₆ alkyl-amine selected from the group consisting of: C₀- C₆ alkyl-NH2_{,} C₀-C₆ alkyl-NHR2, C₀- C₆ alkyl-NR₂ R₃, wherein independently R₂ and R₃ are -H or C₀- C₆ alkyl, and R₂ is C₀ - C₆ alkyl. Particularly preferred alkyl groups are methyl, ethyl, propyl or butyl groups, which can be linear or branched. A preferred alkyl group is methyl. Preferably, R₁ is methyl, ethyl or propyl, or combinations thereof

Preferably, the amine is dimethyl or diethyl amine.

Suitably, particularly preferred monomer are: poly(ethylene glycol) methacrylate, N-(2-hydroxypropyl)methacrylamide., 2-dimethylaminoethyl prop-2-enoate, 2(dimethylamino ethyl) methacrylate or 2(dimethyleamino ethyl) acrylate, although other monomers having vinyl and amine groups can be used.

Suitably, the ratios of divinyl disulfide monomer to co-vinyl monomer may be in the range of from 1:99 to 99:1. A preferred polymer with some knotting has a ration of 10:90.

In a preferred embodiment, the initiator molecule have general structure:

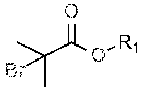

wherein R₁ may be C₁- C₆ alkyl. Preferably, R₁ is methyl, ethyl or propyl. Particularly preferred alkyl group is ethyl.

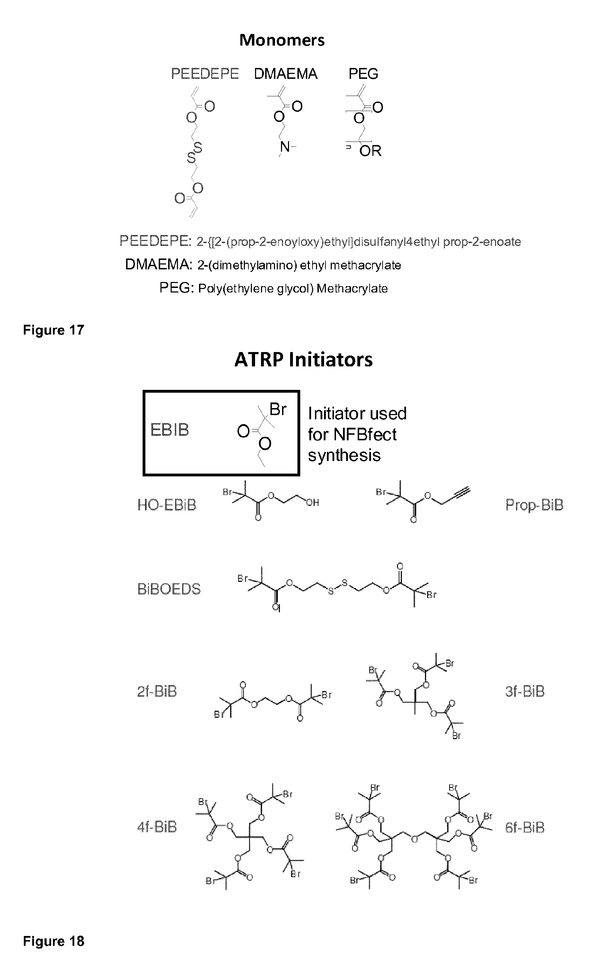

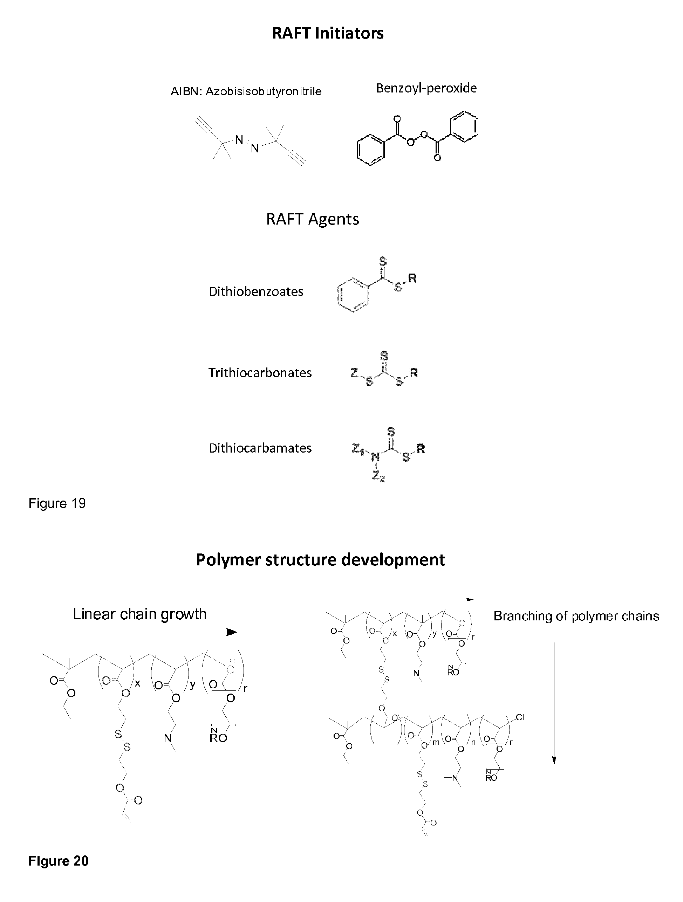

Other suitable initiators (and polymerisation components for RAFT, ATRP and DE-ARTP) are shown in Figures 18 and 19. The skilled person will appreciate the conditions required for such processes.

Preferably, the ratios of initiator to divinyl disulfide monomer is in the range of from 1:1 to 99:1 (all ratios are referenced to the disulfide monomer). One specific example, the ratio is 4:1 disulfide: initiator.

Suitably, a preferred initiator is: 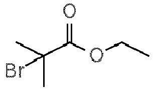 ethyl 2-bromo-2-methyl-propanoate.

This particular initiator compound can be subjected to co-polymerisation via a self-condensing vinyl polymerization process involving vinyl monomer 2-dimethylaminoethyl prop-2-enoate having structure:

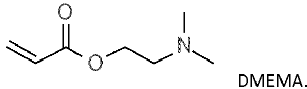

In a particularly preferred embodiment, in the method of the invention, a vinyl monomer compound is selected from the group of general structures consisting of: 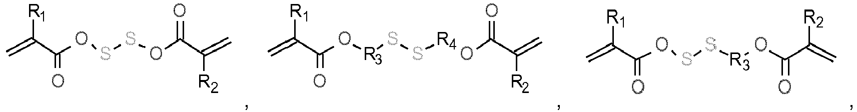 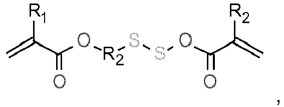 in which, independently, R₁, R₂, R₃ and R₄ can be C₀ - C₆ alkyl, and
a second vinyl monomer compound is selected from compounds having general structure: 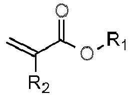 wherein R₁ is C₁- C₆ alkyl-amine selected from the group consisting of: C₀- C₆ alkyl-NH_{2,} C₀-C₆ alkyl-NHR₂, C₀- C₆ alkyl-NR₂ R₃, wherein independently R₂ and R₃ are -H or C₀- C₆ alkyl, and R₂ is C₀ - C₆ alkyl; and
wherein the initiator is selected from a compound having general structure: 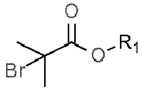 wherein R₁ may be C₁- C₆ alkyl.
In a preferred embodiment, the multi-vinyl monomers are a mixture of 2-(dimethylamino) ethyl methacrylate (DMAEMA) and 2-{[2-(prop-2-enoyloxy)ethyl]disulfanyl-4-ethyl prop-2-enoate (PEEDEPE).Suitably, the polymerisation initiator is EBIB.

Preferably, the catalyst of the invention is a mixture of CuCl/CuCl₂.

Desirably, the terminating group of the polymer of the invention is provided by conjugate addition (Michael Addition) of diaminopropane to the vinyl group.

Preferably, the self-condensing vinyl polymerization process is a deactivation atom transfer radical polymerisation (DE-ATRP), ATRP or a RAFT process.

Suitable RAFT agents function as terminating groups in a RAFT polymerisation - such agents are shown in Figure 19. The ratios of initiators and RAFT agents to the divinyl disulfide monomer can range from 1:1:1 to 1:99:1 (RAFT initiator: disulfide: RAFT agent).

### Brief Description of the Drawings

Figure 1 shows expression of collagen in keratinocytes after transfection with the polymer of the invention in the form of a polyplex ("polymer of the invention");

Figure 2 shows the superior transfection efficiency in a variety of cell types compared to PEI and Lipofectamine^{™}2000 [Polymer/DNA @ optimal w/w];

Figure 3 demonstrates the reduced toxicity of the polymer of the invention;

Figure 4 demonstrates that the polymer of the invention degrades within minutes;

Figure 5 is a schematic of the polymer synthesis and purification using the DE-ATRP process;

Figure 6 is a more detailed schematic of the polymer synthesis and purification using DE-ATRP;

Figure 7 shows GPC trace for the synthesis of the polymer and growth;

Figure 8 indicates size and charge measurements of the polymer of the invention obtained by zeta potentials;

Figure 9 demonstrates complexation capability of DMAD at different DNA/polymer weight ratios.

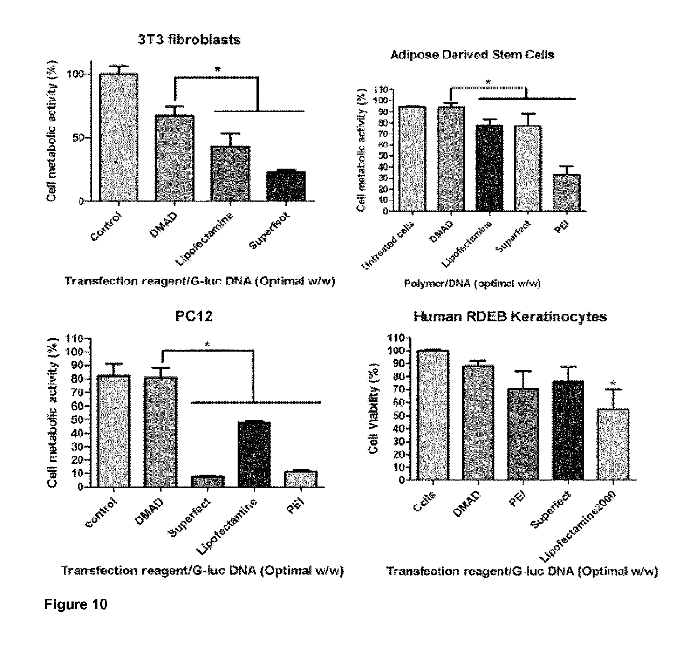

Figure 10 demonstrates results of cell viability studies in various cells.

Figure 11 shows Luciferase expression activity analysis of DMAD demonstrated the polymer's capability to efficiently deliver and release DNA into the cell;

Figure 12 comparative Luciferase expression results after transfection with various transfection agents;

Figure 13 shows the percentage of transfected keratinocytes with DMAD compared with other commercially available reagents.

Figure 14 demonstrates expression of type VII collagen after transfection with various transfection agents;

Figure 15 illustrates an immunoblot of RDEB keratinocytes transfected with various transfection agents;

Figure 16 is a schematic of a typical hyperbranched polymer of the invention;

Figure 17 the chemical structures of the primary monomers used for the synthesis of DMAD;

Figure 18 list of the chemical structures of the initiators that could be used for ATRP reaction for the synthesis of DMAD;

Figure 19 list of the RAFT initiators and RAFT agents that could be used for the RAFT synthesis of such polymer as DMAD;

Figure 20 Polymer structure development from linear to branched;

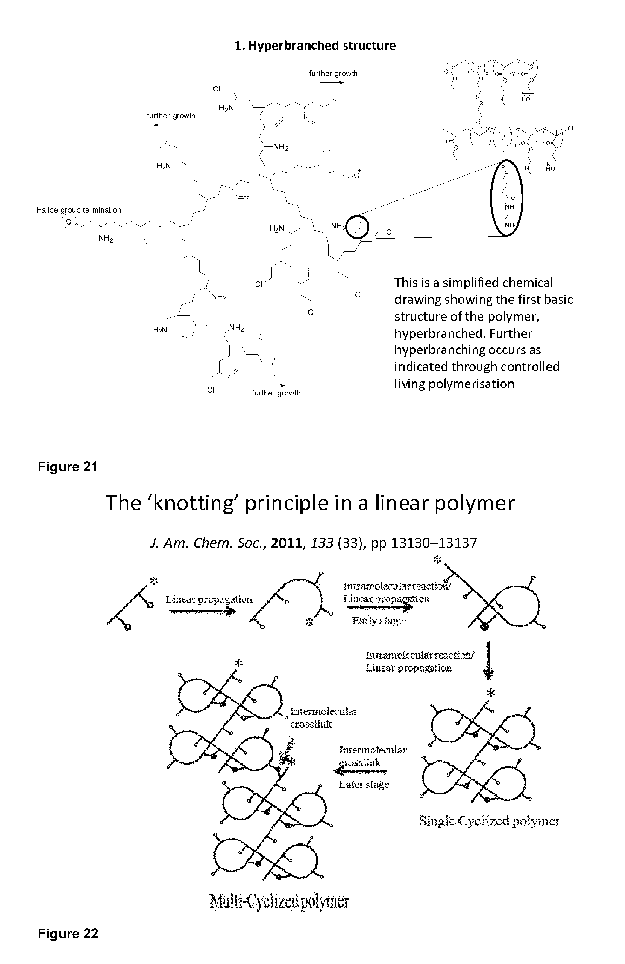

Figure 21 simplified hyperbranched structure of the polymer;

Figure 22 the principle of knotting/cyclising within a linear polymer;

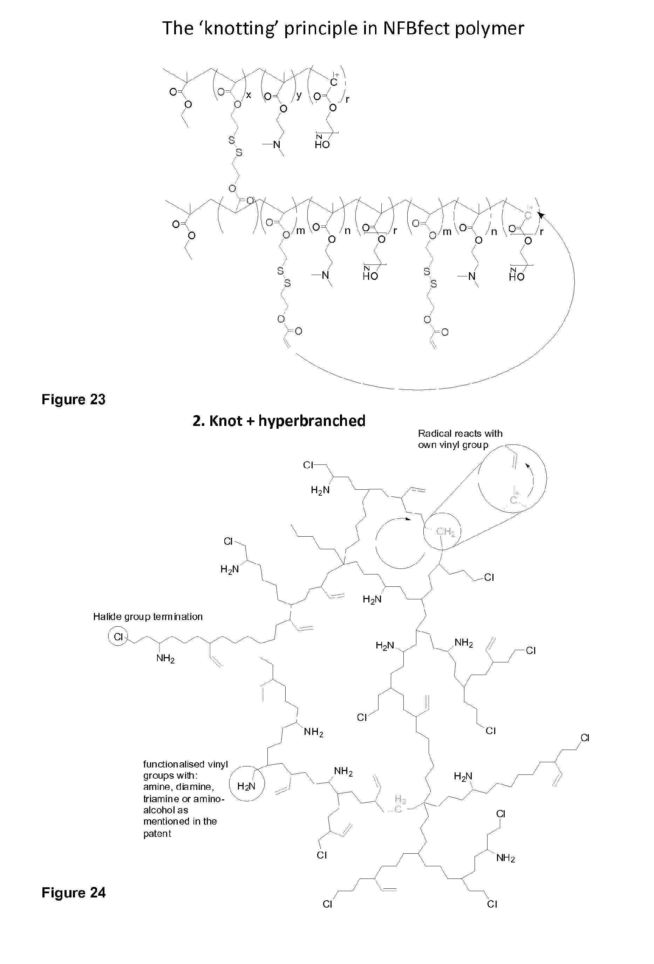

Figure 23 the principle of knotting/cyclising within the DMAD polymer; and

Figure 24 the final simplified structure of DMAD which includes both knotted and hyprebranched structures.

### Detailed Description of the Invention

The inventors have provided a platform for a non-viral, synthetic polymer based approach for gene delivery that overcome the current challenges associated with non-viral gene therapy. The approach of the invention uses Deactivation-Enhanced Atom Transfer Radical Polymerisation (DE-ATRP) to control the rate of polymer synthesis to achieve a specific molecular weight polymer in a one-pot reaction. The polymer of the invention as described herein was developed using this approach. Preferably, the polymer is hyperbranched.

Hyperbranched polymers belong to a class of synthetic tree-like macromolecules called dendritic polymers. They are polymers with densely branched structure and a large number of end groups. Dendritic polymers include dendrimers which have completely branched star-like topologies and hyperbranched polymers which have imperfectly branched or irregular structures. Both dendrimer and hyperbranched polymer molecules are composed of repeating units emanating from a central core. The core is characterised by its functionality, which is the number of chemical bonds through which it can be connected to the external parts of the molecule. The functionality of the core is normally three (e.g. amine) or four (e.g. ethylenediamine). Through the bonds of the core, the layers of linear units (single monomers or linear chains) are attached to the core and each of these arms is terminated with the multifunctional branched unit. Larger molecules are created by adding shells of linear units to the end groups of the layer beneath. If all of these units are attached to the molecule perfectly, a dendrimer is formed. In contrast, the absence of any of these units in the molecule will result in a hyperbranched polymer structure.

DNA complexation, cell viability and cell transfection results show promise for these newly synthesised polymers. Polyplexes (polyelectrolyte complexes of nucleic acids with polycations) have been investigated as promising delivery vectors for an assortment of therapies. Most notably, polyplexes with the ability to alter their behaviour and properties to respond to stimuli or environmental changes insure a substantial improvement of the performance of the delivery process. They depict a promising non-viral alternative for the delivery of many nucleic acid therapies and can be optimized and fine tuning to provide the optimal molecular weight, amine/acrylate and polymer/DNA ratios, and particle size for each cell and treatment type. These polymers in combination with the advances in plasmid design will have a profound effect on the non-viral gene therapy field.

Gene delivery vectors have technical design criteria such as packaging of large DNA plasmids, protection of DNA, serum stability, specific cell targeting, internalisation, endolysosomal escape and nuclear localisation with minimal toxicity to name a few. Other criteria include economic and scale-up production factors: ease of administration, ease of fabrication, inexpensive synthesis, facile purification and safety. A much-studied cationic polymer for gene delivery is linear poly (DMAEMA) (PDMAEMA) containing tertiary amine groups for complexing DNA5. However, most of the cationic based polymers that are currently used in research lack the high efficiency displayed The cationic polymer described herein help with achieving that goal. Since cationic polymers seem to work differently with different cell types, our system is flexible enough to allow for small changes to the polymer to accommodate the required clinical target. Recent results showed protein expression in a number of cells using our three newly synthesised polymer (DMAD). This polymer formed complexes with DNA in the nanometre scale (<500nm) which were efficiently taken up by the cell. The excess positive charge of the nano-particles meant that they were capable of escaping the endosome by the "proton sponge" effect and degrading in the cytoplasm allowing for release of the DNA and subsequent expression of the protein. The polymers showed higher transfection capability (and higher protein expression) after transfection compared to current gold standard polymers (PEI, LipofectamineTM2000, superfect) with less cytotoxicity and higher DNA condensation capability. DMAD has better biodegradability due to the additional cleavage of the disulfide bond by cellular enzymatic activity and this is demonstrated by lowered cytotoxicity and higher transfection rates of 3T3 fibroblasts and collagen type VII null RDEB keratinocytes. Elevated protein expression is seen in hard to transfect cells such as Neu7.

The present invention involves development of polymers for the investigation of non-viral gene delivery in a number of clinical targets by the use of non-viral vectors for the efficient delivery of genes to cells for gene therapy. Clinical targets include nucleus pulpous, Parkinson's, cardiovascular disease and Recessive Dystrophic Epidermolysis Bullosa.

Furthermore, the invention provides a synthesis platform suitable for the production of specifically designed cationic polymers that are demonstrated to deliver improved transfection efficiency and cytotoxicity performance when compared to currently available gold standard gene delivery reagents. The polymer of the invention (designed herein as **'NFBfect')** is an example of one such polymer. The methods provided herein are capable of synthesizing polymers with impressive biodegradable properties [intracellular] and the specific addition of modifiable functional groups, making antibody (or indeed moieties of a variety of other applications) attachment a genuine prospect.

In particular, the Inventors have demonstrated the synthesis and characterisation of hyperbranched or linearly branched cationic polymers for the effective gene delivery for the treatment of genetic related diseases.

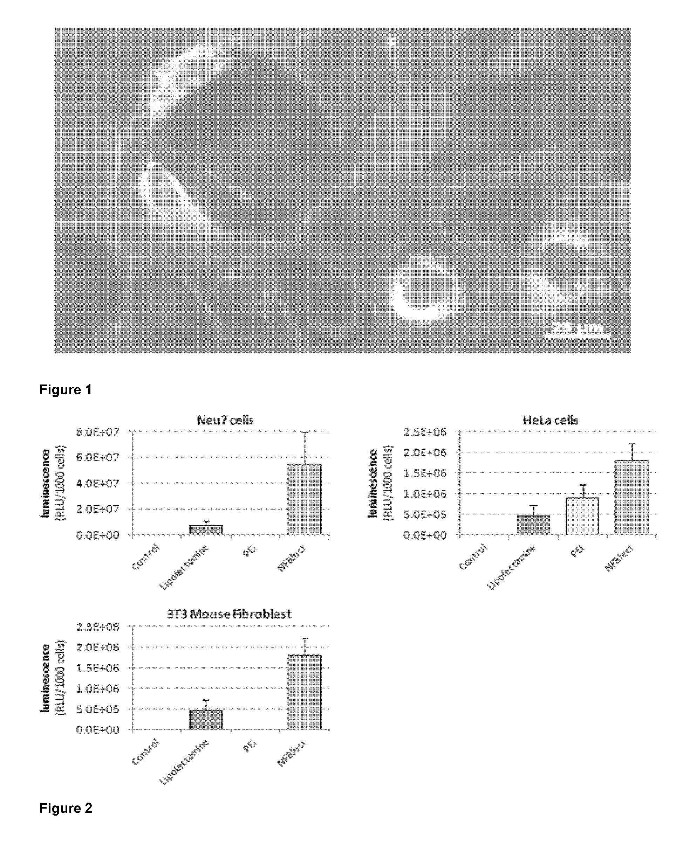

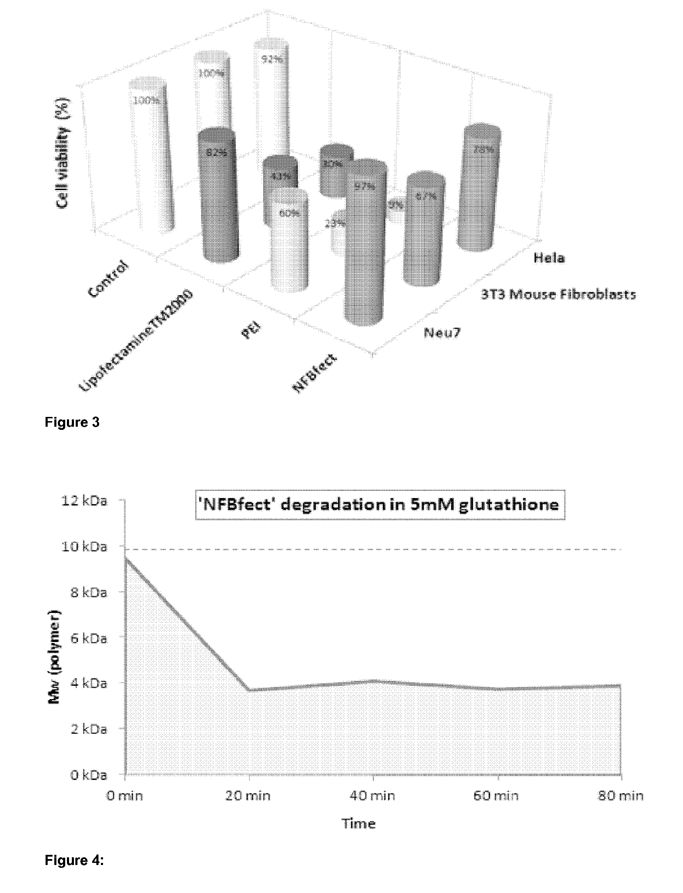

In comparison to commercially available transfection agents, the polymers of the invention have:
**1. Superior Transfection Efficiency -** transfection efficiency in a variety of cell types demonstrates superior luciferase expression compared to PEI and Lipofectamine^{™}2000 [Polymer/DNA @ optimal w/w] (See Figure 2);
**2. Reduced Toxicity -** aided by superior biodegradability when placed in physiological conditions (Figure 3). Under physiological conditions (glutathione), NFBfect degrades within minutes (Figure 4); and
**3. Function Group Modification and Gene Therapy Potential.**

Polymer Synthesis & Characterisation -Copolycondensation reactions are not suitable for forming hyperbranced copolymers as vinyl monomers cannot be polymerized by these methods. However, SCVP can be used to produce such hyperbranched polymers. Typically, a vinyl monomer of structure AB can be used, where A is a vinyl group and B is a functional group that can be converted into an initiating group B* by an external stimulus. This species is known as an inimer. The activated species can be a "living" free radical, an electrophilic cationic moiety or a carbanion. Inimers often comprise halogen groups, whereby activation occurs by removing the halogen to form either a cation or a radical. Controlled/living radical polymerisation (CRP) is a widely used technique that allows the synthesis of defined polymer architectures through precise control of molecular weights and distributions. The architectures of polymers prepared by the CRP techniques are reported to be the linear, crosslinked, branched/dendritic structures. By precisely controlling the competition between chain growth and reversible chain termination, the polymers can grow slowly and effectively while branching is introduced by the multi-functional vinyl monomers in a controlled fashion and the cross linking reaction is delayed. DE-ATRP allows the simple use of readily available and inexpensive multi-functional vinyl momomers to synthesis soluble hyperbranced polymers with a high degree of branching and controlled chain structure via homo- and copolymerisations. The key to the strategy is to control competition between chain growth and revesible chain termination via a deactivation enhanced method. Thus the polymer chains can grow effectively and brancing is introduced by multifunctional vinyl monomer in a controlled fashion, leading to the formation of hyperbranced polymers rather than cross linked gel.

The non-viral vectors are synthetic polymers that are synthesised by the process of Atom Transfer Radical Polymerisation (ATRP) or Reversible Addition Fragmentation Chain-transfer (RAFT) polymerization to yield hyperbranched or linearly branched polymers.

The polymers are composed of 2-(dimethylamino) ethyl methacrylate (DMAEMA), 2-{[2-(prop-2-enoyloxy)ethyl]disulfanyl4ethyl prop-2-enoate (PEEDEPE, synthesised in the lab and not available commercially), and Polyethylene glycol (PEG) (meth)acrylate.

The DNA condensation is achieved by the cationic amine groups of the DMAEMA, while the reduced cytotoxicity is accredited to the reduction of the disulfide bonds in the PEEDEPE monomer which is also the branching agent.

The PEG monomer adds protection from serum proteins credit to its hydrophilic nature. The polymer was called disulfide hyperbranched polymers. The polymers contain excessive vinyl groups which were terminated by conjugate addition of amine monomers (mainly 1, 3-Diaminopropane).

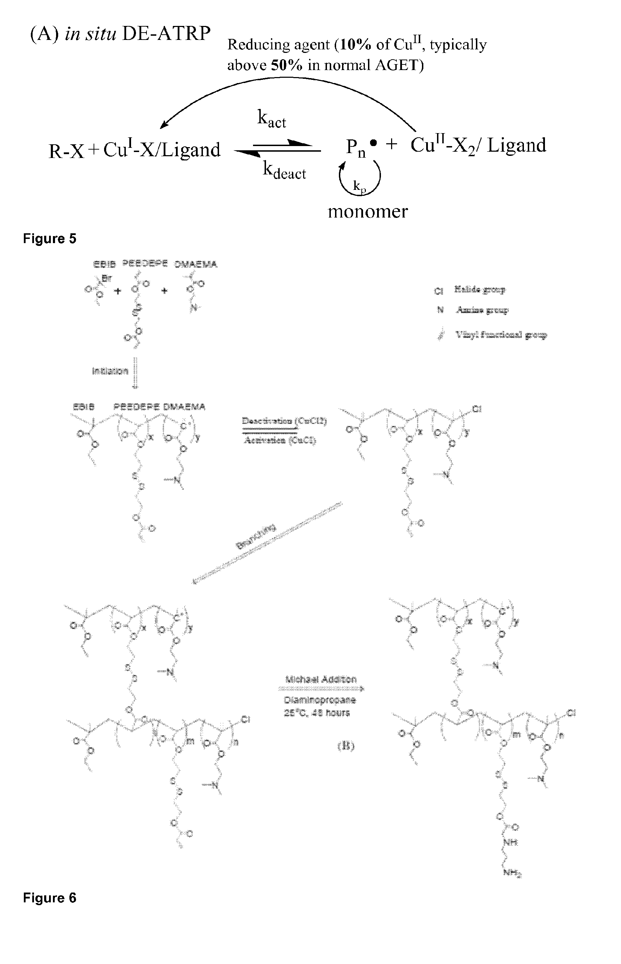

*An example of manufacturing procedure -* The process of synthesising and purifying a preferred polymer of the invention is based on the radical polymerisation known as deactivation-enhanced atom transfer radical polymerisation (DE-ATRP, Figure 5, Figure 6).

The polymer was prepared in acetonitrile (the volume ratios of total monomers to solvent = 1:1) at 60°C with Schlenk line system, where argon was bubbled through the solutions to remove oxygen. Liquids were transferred by means of septa and syringes while under argon. A typical reaction procedure is described: The DMAEMA (5.4g), PEEDEPE (1g), initiator (0.186 g), PMDTA (0.016 g), CuCl₂ (0.013 g) and acetonitrile (7ml) were transferred to a two necked round-bottom flask fitted with stopcocks. Argon was bubbled through the solution for 15 minutes to purge the oxygen. L-Ascorbic acid was added into the flask to start the reaction which was kept in an oil bath at 60°C and stirring at 600rpm. Samples were taken at different time points for GPC analysis to monitor the monomer conversions by comparing the peak areas for monomers and polymers. The reaction was stopped when the desired monomer to polymer ratio was obtained. After polymerization, the obtained polymers were precipitated by adding the solution drop-wise into a large excess of hexane to remove excess DMAEMA and PEEDEPE monomers. The precipitated polymer was dissolved in acetone and passed through an aluminium oxide (Sigma-Aldrich, activated, basic, Brockmann I) glass column to remove the copper.

*Conjugate addition of diamine monomers to form cationic polymer, NFBfect^{™}*-Unreacted vinyl groups of DMA were end-capped by adding 200mg (2mmol, dissolved in water) of the DMA polymer to 40mg (50mmol, dissolved in water) of either 1,3-diaminopropane or ethylenediamine under argon at ambient temperature for 48 hours in the dark. The end-capped polymer (DMA) solution was then protonated to a pH of 5.6 using 1 mol HCL, dialysed against water using 6,000-8,000 *MWO* Spectra/Por® cellulose membrane (Spectrum labs, Medical Supply Co., Dublin 15, Ireland), and freeze dried to obtain a light yellow powder.

**Characterization of the polymer** - Two of the most significant factors that effect polyplex uptake by cells are their cationic charge and hydrodynamic size. The zeta-potential and hydrodynamic size of the nano-particles were determined using multimode measuring equipment that utilises the DLS and charge properties of colloidal particles to determine the particle size and potential respectively. The polymer of the invention forms polyplexes with zeta-potential higher than PEI and LipofectamineTm2000. The polymer of the invention forms polyplexes with average diameter size of less than 200 nm. Advantageously, the high positive charge and small diameter bring about enhanced cell update and internalization of polyplexes. Reduction in polymer size occurs within minutes in intracellular space. This reduction enhances DNA release into the cytoplasm. Reduction is followed by byproduct elimination from the cells which result in enhanced cell viability. Cell viability remains high relative to gold standard transfection reagents (this is seen in 5 different cell types - see Figures).

Transmission electron microscopy (TEM) was used to obtain visual confirmation of particle sizes (figure 5).

The resultant polymer was characterized by gel permeation chromatography (GPC) and proton nuclear magnetic resonance (¹H NMR). Number average molecular weight (Mₙ), weight average molecular weight (M_{w}), and polydispersity (M_{w}/Mₙ) were obtained by GPC (920-LC Liquid Chromatograph, Varian) with a refractive index detector, column heater and evaporative light scattering (ELS) detector supplied by Varian. The columns (300×7.5 mm PolarGel-M Column, two in series) were eluted using DMF and calibrated with poly (methyl methacrylate) standards. All calibrations and analyses were performed at 60 °C and a flow rate of 1 mL/min. ¹H NMR was carried out on a 400 MHz JEOL NMR with DELTA processing software. The chemical shifts were referenced to the lock (CD3)2CO.

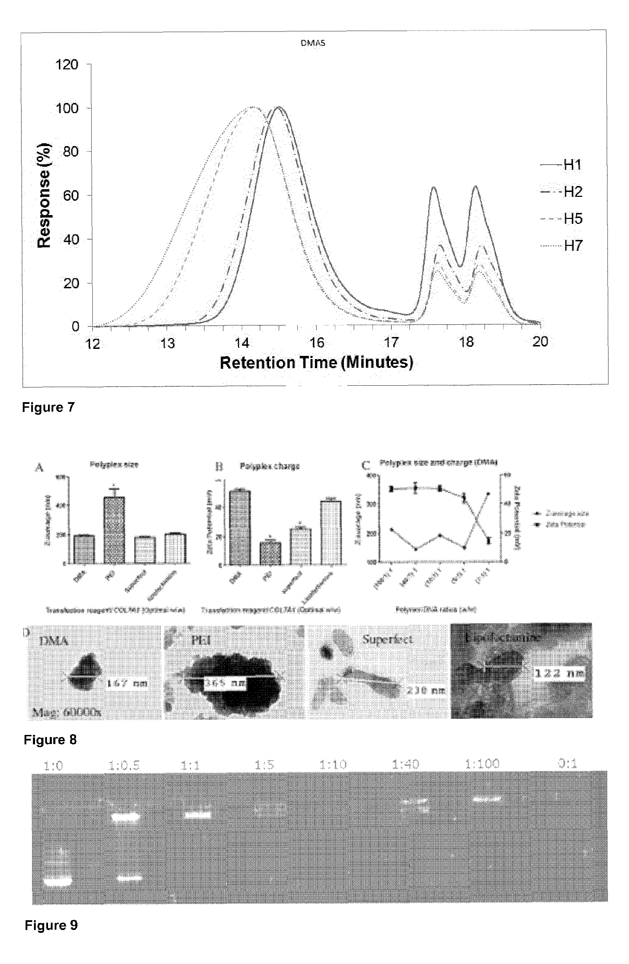

**Co-polymer Synthesis: -** The polymer (DMAD) was successfully synthesised using DE-ATRP in controlled conditions to obtain high molecular weight polymers with low polydispersity (PDI ∼1.2) (see Figure 7, showing the GPC trace for the synthesis of DMAD showing the peak shift (increase in molecular weight) and increased height (monomer conversion) over time. Samples were taken for analysis after 1 hour (H1), 2hours (H2), 5 hours (H5), and 7 hours (H7).

Size and charge measurements of poyplexes were obtained from Zetasizer and TEM (Figure 8). (A) DMA/*COL7A1* polyplex sizes and equivalent Zeta-potential at various ratios. As the polymer/DNA weight ratio (w/w) increases, a proportional decrease in polyplex size is observed. (B) Zeta-potential of polyplexes formed from various transfection agents showing DMA polymer with the highest charge (DMA/*COL7A1* w/w =10:1) compared to other transfection agents. (C) polyplex sizes corresponding to (B). (D) TEM images of DMA, PEI, superfect and lipofectamine polyplexes.

Results are displayed as the means and standard deviations from 3 values (n=3, ±S, for statistical significance *P<0.05).

Size and charge measurements of poyplexes as obtained from Zetasizer and TEM are shown in Figure 4. (A) DMA/*COL7A1* polyplex sizes and equivalent Zeta-potential at various ratios. As the polymer/DNA weight ratio (w/w) increases, a proportional decrease in polyplex size is observed. (B) Zeta-potential of polyplexes formed from various transfection agents showing DMA polymer with the highest charge (DMA/*COL7A1* w/w =10:1) compared to other transfection agents. (C) polyplex sizes corresponding to (B). (D) TEM images of DMA, PEI, superfect and lipofectamine polyplexes.

Results are displayed as the means and standard deviations from 3 values (n=3, ±S, for statistical significance *P<0.05).

**Polymer/DNA complexation -** The ability of the polymer to complex plasmid Gaussia Luciferase (G-Luc) DNA was then assessed by gel electrophoresis. Polymer/plasmid solutions were made in phosphate buffered saline (PBS) at various weight ratios by adding 10µg to varying concentrations of polymer. These were left gently shaking for 1 hour to form complexes before analysis. An agarose gel (10% agarose in Tris-borate-EDTA (TBE) buffer, with SYBR®Safe DNA stain) was made up for all polymers tested. 5µl of each polymer/plasmid solution (DNA concentration of 50 µgml-1) were added along with 5µl loading dye to each well and subjected simultaneously to 80mV for up to 2 hours (Fig 4).

**Complexation Capability** - Complexation capability of DMAD at different DNA/polymer weight ratios is shown in Figure 8. Even at a ratio of 1:0.5, it is clear that most of the DNA is complexed with the polymer as the DNA becomes trapped only free DNA continues to flow towards the positive charge. As the amount of polymer is increased, no free DNA is observed, in fact at a ratio of 1:40 the complex begins to travel in the opposite direction towards the negative electrode indicating that the complex is more cationic than at lower ratios.

**Cell viability -** Cell viability studies for DMAD show lower cytotoxicity in most cell types compared with current commercial polymer vectors. Results of cell viability studies of DMAD, in mouse 3T3 fibroblasts, rat adipose derived stem cells, PC12 pheochromocytoma and human RDEB keratinocytes at optimal polymer/DNA weight ratios are shown in Figure 9. Controls (PEI, Superfect® and LipofectamineTM) also have an optimal w/w ratio. (n=5)(±S)(P<0.05).

**Cell Transfection:** - Luciferase expression activity analysis of DMAD demonstrated the polymer's capability to efficiently deliver and release DNA into the cell. Gaussia luciferase expression results indicate that DMAD is capable of efficiently transfecting type VII collagen-null keratinocytes (RDEBK), rabbit-adipose derived stem cells (ADSCs), 3T3 fibroblasts and Neu7 astrocytes.

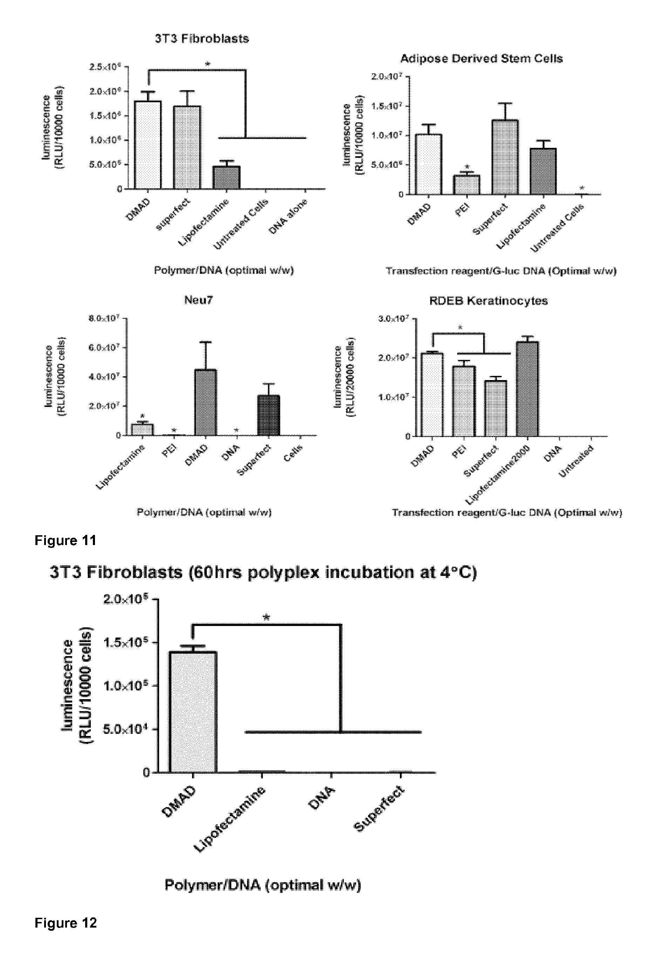

Luciferase expression quantified by luminescence after transfection with various transfection agents including DMAD (see Figure 11). Luciferase activity after transfection with polyplexes incubated in water for 60 hours at 4°C indicate that DMAD forms more stable polyplexes than commercial vectors. Results are displayed at optimal w/w ratios of polymer/DNA. (n=5)(±S)(P<0.001).

**Transfection efficiency analysis** - In order to measure the percentage of transfected cells when using optimal formulations for DMA, PEI, Superfect and lipofetamine, we used the eGFP as a marker gene and measured the gene expression by FACS analysis. Out of a gated 10,000 recoded events, around 10% of RDEBK cell population showed expression for eGFP. Meanwhile, only 4-6% efficiency of transfection was observed in cells transfected with PEI, Superfect and lipofectamine polyplexes. These results demonstrate the capability of the polymer to form small charged particles that are easier to uptake in biophysical conditions. Furthermore, the reduction in polymer size upon cell internalization can also be proven by this method as protein expression depends on the eGFP release from the polymer.

Flow cytometery dot plots (top) and histograms (bottom) of polymer or liposome poyplex transfected RDEB keratinocytes are shown in Figure 12. Efficiency of transfection was measured by counting the number of eGFP transfected cells using BDFACSCanto equipment. DMA transfected cells showed the highest percentage of total transfected cells compared to other transfection agents.

***COL7A1* expression after transfection -** Indirect immunoflourescence staining was carried out on RDEB Keratinocytes transfected with polyplexes carrying the *COL7A1* sequence. Expression of type VII collagen in transfected cells was visualized using an affinity-purified antibody to the NC1 domain of the type VII collagen. Large amounts of the protein were seen in cells transfected with the DMAD polyplexes with some being released into the extracellular matrix (figure 9a). Transfection data of collagen type VII null keratinocytes, using the polymer of the invention carrying the GFP plasmids, showing about 10% of cells are expressing the green fluorescent protein. The number of cells expressing the green fluorescent protein are two folds higher with the polymer of the invention than in groups transfected with commercial reagents.

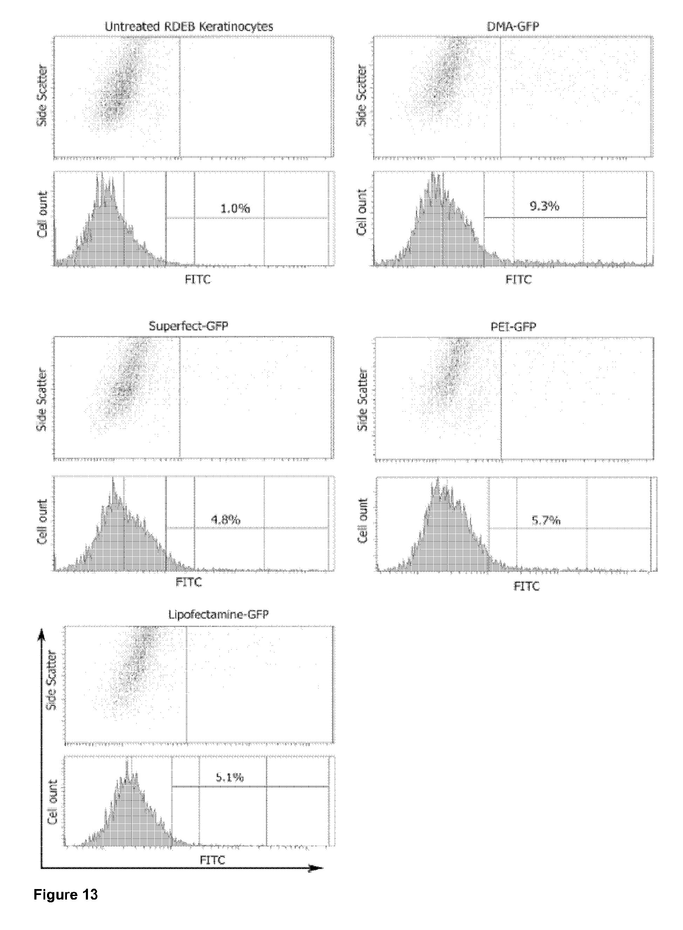

Expression of type VII collagen (Green) in type VII collagen-null RDEB keratinocytes (RDEBKs) after (a) DMAD, (b) PEI, (c) lipofetamine and (d) naked *COL7A1* transfection is shown in Figure 13. DMA transfected cells show the highest amount of type VII collagen expression (5%, as analyzed using threshold on ImageJ). Cell identification was achieved by counterstaining with rhodmaine-phalloidin (red) and DAPI (blue). Areas of significant expression are indicated with arrows. Collagen type VII expression in collagen VII null human RDEB keratinocytes was also analysed by western blot after transfection. DMAD transfected cells showed the highest amount of collagen type VII in RDEB keratinocytes in immunoblot.

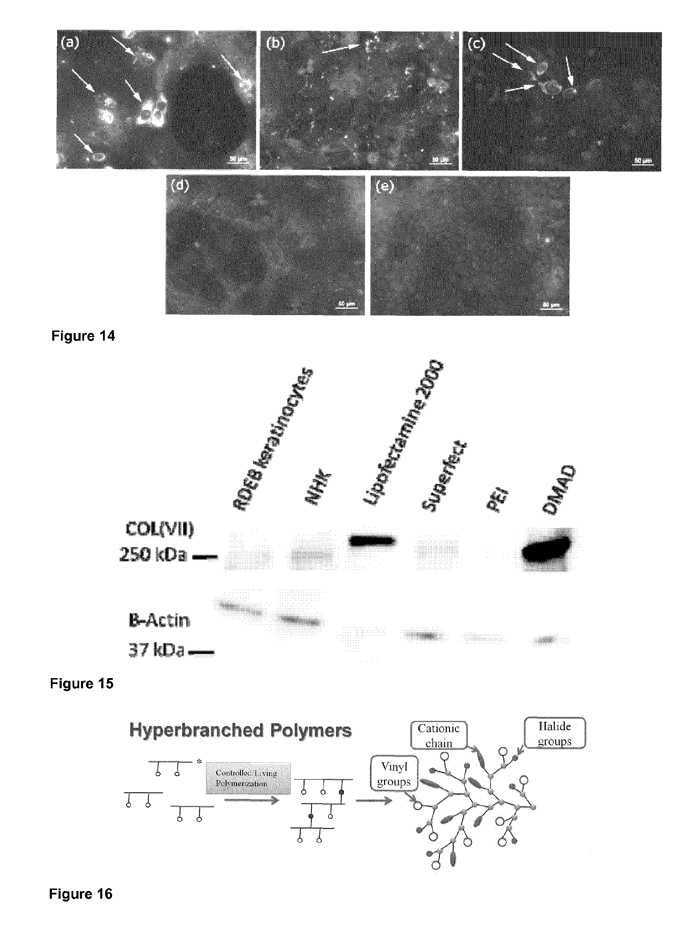

Immunoblot of RDEB keratinocytes transfected with various transfection agents is shown in Figure 14. Positive controls include NHK, Lipofectamine2000, Superfect and PEI, negative control is untreated RDEB keratinocytes.

### Conclusions

In conclusion, the inventors have developed a biodegradable cationic polymer from DE-ATRP and end-capped with diamine monomers by conjugate addition. The polymer shows significantly higher transfection and cell metabolic activity over conventional dendrimer and liposome based transfection agents. Efficient transfection and protein expression are clearly demonstrated using this polymeric vector due to its high cationic charge and disulfide content that degrade upon cell internalization.

## Claims

1. A polymer for transfecting a nucleic acid into a cell, the polymer comprising a plurality of branches, wherein at least one of the branches comprises: at least one disulfide group, and/or at least one vinyl group, which may an unsubstituted vinyl or a functionalized vinyl group, and/or combinations thereof.

2. The polymer of any preceding Claim wherein each of the plurality of polymeric branches comprises an alkyl based chain from which further alkyl based chains split off to form further branches.

3. The polymer of Claim 1 or 2 wherein at least one of the plurality of polymeric branches comprises at least one ionisable amine, diamine, tri-amine or amino alcohol functionality.

4. The polymer of any preceding Claim wherein at least one of the plurality of polymeric branches terminates with a hydroxyl functionality.

5. The polymer of Claim 1 or 2 wherein at least one of the each of the plurality of polymeric branches terminates with a halide selected from the group consisting of: F, Cl, Br and I.

6. The polymer of any preceding claim in which the polymer is hyperbranched.

7. The polymer of any preceding claim in which the polymer is a knotted polymer which can be single cyclized or multi-cyclized.

8. The polymer of any preceding claim in which the polymer is polydisperse.

9. The polymer of claim 1 wherein the polymer is hyperbranched and comprises
a plurality of branched alkylene chains,
wherein at least one of the alkylene branches comprises at least one disulfide group in the alkylene chain, and the alkylene sidechain is further substituted with at least one vinyl group or vinyl functionality; and
wherein at least one of the alkylene branches terminates with at least one of a carbocation, a halide, an ionisable amine, diamine and a hydroxyl group.

10. The polymer of any preceding claim wherein the vinyl group or vinyl functionality is a methacrylate group which can be unsubstituted or substituted with a linear or branched C₁₋₆ alkyl.

11. Use of a polymer as defined in any one of claims 1 to 10 as a nucleic acid or a drug delivery agent or as a transfecting agent in non-viral gene delivery or as a polyelectrolyte for complexing nucleic acid or in an analytical application in bio-imaging techniques for tagging proteins/nucleic acids inside the cell.

12. Polymer for use as defined in claim 11, wherein the agent is for the treatment of a disease that can be treated by genetic or nucleic acid manipulation.

13. A process for synthesising a hyperbranched polymer comprising polymerising a divinyl disulfide monomer with other optional polymerisable monomers and/or initiators under suitable polymerisation conditions to effect polymerisation of the monomer by a living polymerisation process.

14. The process of claim 12 or 13 wherein the divinyl disulfide monomer is selected from the group consisting of: 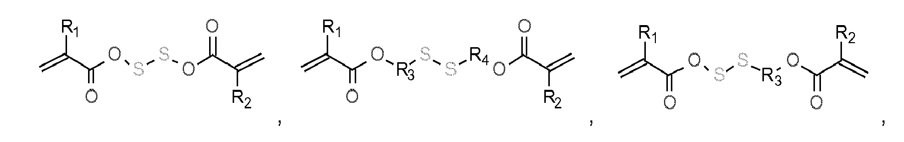 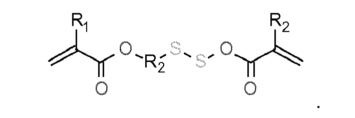 in which, independently, R₁, R₂, R₃ and R₄ can be C₀ - C₆ alkyl.

15. A method of transfecting an cell, comprising contacting the cell with a transfection agent comprising a hyperbranched polymer comprising a plurality of disulfide groups and a plurality of vinyl groups or vinyl functionalities, wherein the step of contacting the least one comprising nucleic acid and a polycationic compound transfects the cell with the nucleic acid.
